# EUROPEAN PATENT SPECIFICATION

(11) **EP 3 722 043 B1**
(45) Date of publication and mention of the grant of the patent: **01.06.2022**
(21) Application number: 18905159.2
(22) Date of filing: 13.03.2018
(51) Int. Cl.: B23K 26/06, A61C 3/00, B23K 26/082, A61B 18/20, A61C 1/00, B23K 26/10

(54) **MULTI-DEGREE OF FREEDOM LASER PROCESSING DEVICE**
LASERBEARBEITUNGSVORRICHTUNG MIT MEHREREN FREIHEITSGRADEN
DISPOSITIF DE TRAITEMENT AU LASER À MULTIPLES DEGRÉS DE LIBERTÉ

(30) Priority: 09.02.2018 CN 201810135368
(43) Date of publication of application: 14.10.2020
(73) Proprietor: Beijing University School of Stomatology, Beijing, 100081 (CN)
(72) Inventor: YUAN, Fusong, Beijing, 100081 (CN); LV, Peijun, Beijing, 100081 (CN)
(74) Representative: Bjerkéns Patentbyrå KB (Stockholm)
(86) International application number: PCT/CN2018/078792
(87) International publication number: WO 2019/153413

(56) References cited:
- CN-A- 102 715 956
- CN-A- 104 546 151
- CN-A- 106 424 286
- CN-B- 103 800 083
- CN-U- 206 343 764
- CN-U- 207 930 150
- JP-A- H1 094 547
- JP-A- H09 173 354
- JP-A- 2014 061 214
- US-A1- 2002 069 819
- US-B1- 6 438 305

## Description

### TECHNICAL FIELD

The present invention relates to the medical field or the industrial field, and in particular to a multiple degrees of freedom (Multi-DOF) laser processing device.

### BACKGROUND

In a narrow cavity such as human mouth or throat, a large number of repeated and laborious micromanipulations are performed manually. Visual perception and hand positioning errors are inevitable, which causes low precision of clinical dental preparation, and prevents operational process specifications and relevant standards to be achieved. Dentist and otolaryngologists maintain almost the same posture throughout procedure, which is tiresome. Moreover, emotional fluctuation, fatigue and lack of practice may cause iatrogenic damages. Noise and mechanical vibration generated by the high-speed turbine used in dental therapy may cause discomfort to the patient.

Existing medical robots assisting surgical procedures fall into two broad categories: master-slave control and automatic control. The *DaVinci* system is representative of master-slave control, and has been used in clinical practice and is suitable for a variety of operations. *Skilled Hand* is a domestically-developed master-slave robot under clinical experiments. Automated control robots have been clinically used in orthopedics and neurosurgery.

The existing surgical robotic systems can improve surgical quality to a certain extent, reduce doctors' fatigue and eliminate the vibrations caused by hand. However, the following limitations and problems exist: First, no robotic system can achieve high-precision, synchronized cutting of all hard and soft tissues such as teeth, jawbone and cartilage at the same time; Second, all surgical robotic systems are master-slave control, i.e., they can only be used under the control of a doctor and has no intelligent planning or autonomous operation capability.

In order to overcome the limitations above, patent application No. CN10546151A discloses an automated dental preparation method and apparatus using a numerical controlled laser, which can achieve a three degrees-of-freedom (3DOF) automatic dental preparation, avoiding the discomfort caused by the noise and mechanical vibration from the high-speed turbine. However, this 3DOF motion cannot achieve high-precision cutting of all parts and tissues inside the mouth or throat, cannot be extended to other medical fields such as ophthalmology or orthopedics, and does not meet the need for a wide-range, versatile processing device.

Patent application with publication No. US2002/0069819A1 discloses a process for producing a wear-resistant cylinder bearing surface, including rotating a laser about its longitudinal axis and simultaneously advancing the laser in a direction of the longitudinal axis which is coaxial with a cylinder of a crankcase.

Patent application with publication No. JP 410094547 discloses a medical or dental laser device. A first light guide is extended longitudinally to the laser device through the supply line and the link part and a second light guide is arranged at a front end area of the laser device. The second light guide is rotatably mounted and is configured to be driven by the rotational drive means during the functional operation of the laser device.

Patent application with publication No. CN103800083A (basis for the preamble of claim 1) discloses a miniature automatic dental-preparation cutting device in oral cavity. The device can achieve three degrees of freedom by controlling two degrees of freedom of the galvanometer through the oscillating motor and one degree of freedom of the lens movement. However, this 3DOF motion cannot achieve high-precision cutting of all parts and tissues inside the mouth or throat, cannot be extended to other medical fields such as ophthalmology or orthopedics, and does not meet the need for a wide-range, versatile processing device.

### SUMMARY OF INVENTION

The present invention provides a multiple degrees of freedom laser processing device, which can solve the problem in relevant technologies that the numerical controlled laser-based automated dental preparation method and apparatus can only achieve a three degrees-of-freedom motion and thus is limited in its effective range and does not meet the need for a wide-range, versatile processing device.

According to the present invention, a multiple degrees of freedom (Multi-DOF) laser processing device is defined in claim 1, including: a base, and a three degrees of freedom (3DOF) laser processing unit and a light guiding tube positioned on the base, the light guiding tube having an end provided with a first reflector mount, the 3DOF laser processing unit being configured to adjust a focal length of a laser to adjust a focal plane of a laser cutter and control movement of the laser cutter on the focal plane, the Multi-DOF laser processing device further comprises: a rotating motor and a first transmission mechanism positioned on the base, wherein,
the light guiding tube is rotatably positioned on a light guiding tube frame that is connected to the base; the light guiding tube is drivable by the rotating motor via the first transmission mechanism, to rotate on the light guiding tube frame under control.

Optionally, the first transmission mechanism comprises: a driving wheel positioned on an output shaft of the rotating motor, a driven wheel positioned on the light guiding tube, and a transmission component for transmitting the rotation of the driving wheel to the driven wheel.

Optionally, the driven wheel has a diameter greater than that of the driven wheel.

According to the present invention,
the Multi-DOF laser processing device further comprises: a linear motor and a second transmission mechanism positioned on the base, and
the first reflector mount is hinged to the end of the light guiding tube;
the first reflector mount is drivable by the linear motor via the second transmission mechanism to rotate around an axis of the hinge under control.

Optionally, the second transmission mechanism comprises: a slide sleeve, a first bearing, a slide rod sleeve, a bearing retaining ring, a slide rod and a linkage mechanism, and
the slide rod sleeve is fixed to the bearing retaining ring; an inner surface of the slide rod sleeve and an inner surface of the bearing retaining ring are both fitted to the light guiding tube with a clearance;
an inner surface of the first bearing is fitted to an outer surface of the slide rod sleeve with a clearance;
a plurality of axial slide rails are distributed on an outer surface of the slide sleeve, and the outer surface of the slide sleeve and the plurality of slide rails are fitted to the light guiding tube frame with a clearance; an inner surface of the slide sleeve is fitted to an outer surface of the first bearing with an interference;
the slide sleeve has a protruding end fixedly connected to a moving rod of the linear motor;
the slide rod has one end fixedly connected to the bearing retaining ring or the slide rod sleeve, and another end hinged to the first reflector mount via the linkage mechanism.

Optionally, the light guiding tube is positioned on the light guiding tube frame connected to the base via a second bearing, and
an inner surface of the second bearing is fitted to an outer wall of the light guiding tube with an interference;
an outer surface of the second bearings is fitted to the light guiding tube frame with an interference.

Optionally, the base is positioned on a working platform that is controllable to be moved in an axial direction of the light guiding tube.

Optionally, the Multi-DOF laser processing device further comprises: a laser processing monitoring unit, and the laser processing monitoring unit comprises: a CCD camera frame positioned on the base, a CCD camera mounted on the CCD camera frame, a reflector and a beam combiner, and
the beam combiner is positioned at a laser entering end of the light guiding tube so that a surface of the beam combiner is at a certain angle to an axial direction of the light guiding tube; the reflector is positioned on the base and cooperates with the beam combiner so that an auxiliary light irradiated on the processing surface can enter the CCD camera for imaging after reflections by the beam combiner and the reflector.

Optionally, the beam combiner has a laser-facing side coated with a film anti-reflective to laser and an auxiliary light-facing side coated with a film highly-reflective to the auxiliary light.

Optionally, the Multi-DOF laser processing device further comprises an electromechanical control system, the electromechanical control system being electrically connected to the 3DOF processing unit, the rotating motor, the linear motor, the CCD camera and the working platform, to realize control or monitoring function.

The Multi-DOF laser processing device according to the present invention increases the degrees of freedom of laser processing, and solves the problem in relevant technologies that the numerical controlled laser-based automated dental preparation method and apparatus can only achieve a 3DOF motion and thus is limited in its effective range and does not meet the need for a wide-range, versatile processing device.

### BRIEF DESCRIPTION OF THE DRAWINGS

The accompanying drawings described herein are intended to provide a further understanding of the present invention, forming a part of the invention. The exemplary embodiments and descriptions thereof are for illustrative purposes only and shall not be construed as liming the scope of the invention. In the drawings:
FIG. 1 is a structural view of a Multi-DOF laser processing device according to an example, not covered by the present invention;
FIG. 2 is a structural view of a X-Y plane processing structure of a 3DOF laser processing device according to an example, not covered by the present invention;
FIG. 3 is a structural view of a Z-direction focal plane adjustment structure of a 3DOF laser processing device according to an example, not covered by the present invention;
FIG. 4 is an overall structural view of a 2DOF laser adjustment unit according to an embodiment of the present invention;
FIG. 5 is a cross-section view of a part of a 2DOF laser adjustment unit according to an embodiment of the present invention;
FIG. 6 is a schematic diagram illustrating an internal structure of pitch adjustment of a 2DOF laser adjustment unit according to an embodiment of the present invention;
FIG. 7 is an exploded view of a part of a pitch adjustment structure of a 2DOF laser adjustment unit according to an embodiment of the present invention;
FIG. 8 is an exploded view of another part of the pitch adjustment structure of a 2DOF laser adjustment unit according to an embodiment of the present invention;
FIG. 9 is a schematic diagram illustrating an internal structure of roll adjustment of a 2DOF laser adjustment unit according to an embodiment of the present invention;
FIG. 10 is an exploded view of a roll adjustment structure of a 2DOF laser adjustment unit according to an embodiment of the present invention;
FIG. 11 is a structural view of a laser processing monitoring unit according to an embodiment of the present invention;
FIG. 12 is a schematic diagram illustrating 3DOF laser processing according to an embodiment of the present invention;
FIG. 13 is a schematic diagram illustrating pitch adjustment of the 2DOF laser processing according to an embodiment of the present invention;
FIG. 14 is a schematic diagram illustrating roll adjustment of the 2DOF laser processing according to an embodiment of the present invention;
FIG. 15 is a schematic diagram illustrating pitch-plus-roll adjustment of the 2DOF laser processing according to an embodiment of the present invention;
FIG. 16 is a schematic diagram illustrating an optical path for laser processing monitoring according to an embodiment of the present invention.

### DETAILED DESCRIPTION OF PARTICULAR EMBODIMENTS

A variety of features and exemplary embodiments of the present invention will be described below. For a better understanding of the objects, technical solutions and advantages of the present invention, the present invention will be described in detail with reference to the accompanying drawings and embodiments. It should be understood that the specific embodiments described herein are merely illustrative of the invention and shall not be construed as limiting. The present invention can be implemented without some of the details of these specific embodiments. The following description of the embodiments is to be used as examples for a better understanding of the invention.

It should be noted that, in this specification, relational terms such as first and second are used merely to distinguish one entity or action from another, and do not necessarily require or imply any substantial relationship or order between the entities or actions. Furthermore, the terms "comprise", "include" and any other variations are non-exclusive, i.e., a process, method, article or device that comprises a plurality of elements may comprise not only explicitly-listed elements but also some other elements, or elements that are inherent to the process, method, item or device. In the absence of other limitations, defining an element with the phrase "comprising..." does not exclude the case where the process, method, article or device comprises more of the same element.

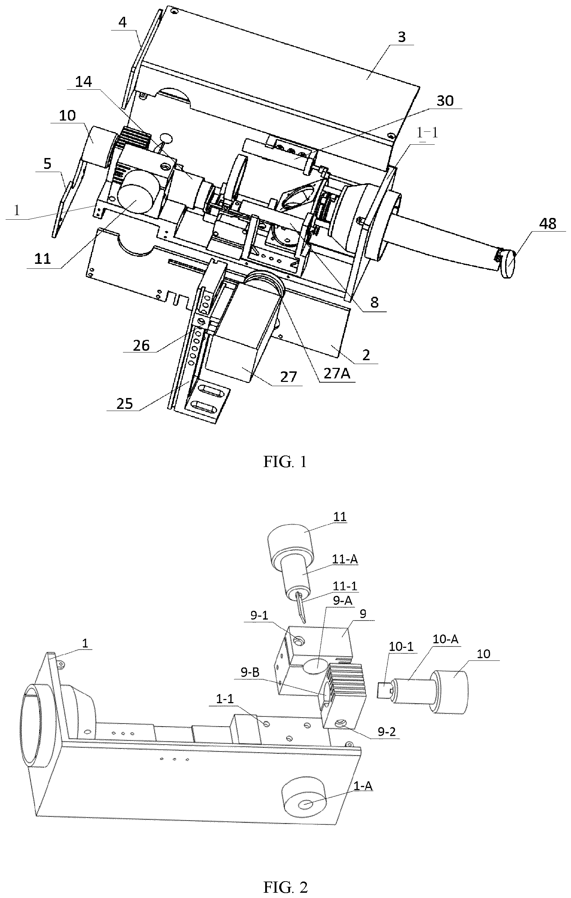

FIG. 1 is a structural view of a Multi-DOF laser processing device according to an example, not covered by the present invention. Embodiments of the present invention will be described below with reference to FIG. 1.

Example 1, not covered by the present invention.

This example provides a 4DOF laser processing device. As shown in FIG. 1, the 4DOF laser processing device includes: a base 1, and a 3DOF laser processing unit (10, 11, 14) and a light guiding tube 37 positioned on the base 1. The light guiding tube 37 has an end provided with a first reflector mount 48. The 3DOF laser processing unit is configured to adjust the focal length of a laser, to adjust the focal plane of the laser cutter (i.e., working plane of the laser cutter) and control movement of the laser cutter on the focal plane. The 4DOF processing device may further include: a rotating motor 8 and a first transmission mechanism positioned on the base 1. Specifically, the light guiding tube 37 is rotatably positioned on a light guiding tube frame 1-1 that is connected to the base 1; and the light guiding tube 37 is drivable by the rotating motor 8 via the first transmission mechanism to rotate on the light guiding tube frame 1-1 under control.

As shown in FIG. 1, in order to protect the internal structure of the device and prevent dust, a housing may be installed over the base 1. The housing structure shown in FIG. 1 includes a side cover 2, an upper cover 3, an upper rear cover 4 and a lower rear cover 5. This housing structure allows for easy disassembly of the housing for maintenance or repair. It will be apparent to those skilled in the art that the above described housing structure is merely a preferred housing configuration.

The 3DOF laser processing unit includes: an X mirror galvanometer 10, a Y mirror galvanometer 11 and a first linear motor 14 and related components. The X and Y mirror galvanometers drive galvo mirrors to realize X-Y plane laser processing; the first liner motor 14 drives a lens to change the focal plane to realize three-dimensional laser processing.

FIG. 2 is a structural view of a X-Y plane processing structure of a 3DOF laser processing device according to an example, not covered by the present invention. The laser beam enters through a light entrance 1-A of the base, and is reflected by a first galvo mirror 10-1 and a second galvo mirror 11-1. The X mirror galvanometer drives the first galvo mirror to rotate, and the Y mirror galvanometer drives the second galvo mirror to rotate, thereby realizing X-Y plane processing. The X mirror galvanometer is fitted to a surface 9-B of a mirror galvanometer mount 9 via a surface 10-A, and fixed by a screw 9-2; the Y mirror galvanometer is fitted to a surface 9-A of the mirror galvanometer mount via a surface 11-A, and fixed by a screw 9-1. The mirror galvanometer mount is connected and fixed to the base by a screw 1-1.

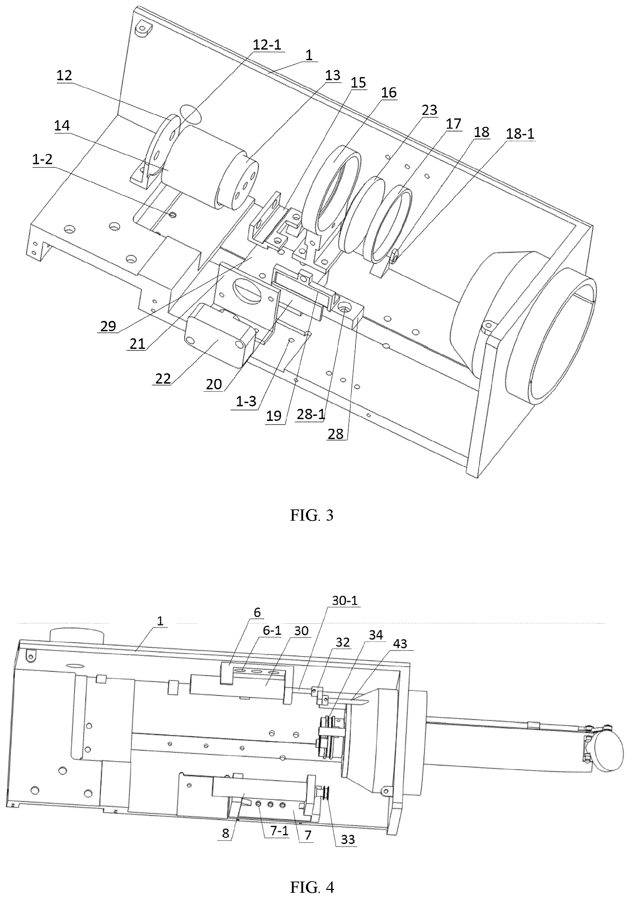

FIG. 3 is a structural view of a Z-direction focal plane adjustment structure of a 3DOF laser processing device according to an example, not covered by the present invention. The stationary coil 14 of the first linear motor is connected to a first linear motor mount 12 by a screw 12-1, and to the base by a screw 1-2. The moving coil 13 of the first linear motor is connected to a lens holder 16 via a motor-lens connecting frame 15. A lens 23 is fixed in the lens holder 16 by a lens fixing ring 17 and a fixing ring stopper 18 with a screw 18-1. The lens holder is installed on a slider 29, which moves along a slide rail 28. The slide rail is connected to the base by a screw 28-1. The first linear motor drives the lens to slide along the slide rail, which changes the position of the lens and thus changes the focal plane of the laser. By using readings from an optical linear encoder 20 as the feedback information, the device can precisely control the sliding displacement of the lens. The optical linear encoder is fixed to the lens holder via an optical linear encoder mount 19. The optical linear encoder has a reading head 22 installed on the optical linear encoder mount 21. The optical linear encoder mount is connected to the base by a screw 1-3.

The 4DOF laser processing device above includes a rotating motor 8 and a first transmission mechanism positioned on a base 1, and accordingly, in comparison with a 3DOF processing device, has a light guiding tube 37 that is rotatable around an axis, which enables the working plane of the laser cutter to rotate around the axis, thereby increasing the degrees of freedom of the laser cutter, expanding the application of the laser processing device and satisfying the need for a versatile processing device.

Optionally, the first transmission mechanism includes: a driving wheel 33 positioned on an output shaft of the rotating motor 8, a driven wheel 34 positioned on the light guiding tube 37 and a transmission component for transmitting the rotation of the driving wheel 33 to the driven wheel 34. Optionally, the diameter of the driven wheel 34 is greater than the diameter of the driven wheel 33.

Example 2, not covered by the present invention

This embodiment provides a 4DOF laser processing device. The device can be basically the same as the 4DOF laser processing device described in Example 1, except that: the present embodiment does not include the rotating motor 8 nor the first transmission mechanism; instead, the present embodiment includes: a linear motor 30 and a second transmission mechanism positioned on the base 1. Specifically, the first reflector mount 48 is hinged to the end of the light guiding tube 37; the first reflector mount 48 is drivable by the linear motor 30 via the second transmission mechanism to rotate around an axis of the hinge under control.

Optionally, the second transmission mechanism includes: a slide sleeve 43, a first bearing 40, a slide rod sleeve 41, a bearing retaining ring 42, a slide rod 44 and a linkage mechanism 45. Specifically, the slide rod sleeve 41 is fixed to the bearing retaining ring 42; the inner surface of the slide rod sleeve 41 and inner surface of the bearing retaining ring 42 are both fitted to the light guiding tube 37 with a clearance; the inner surface of the first bearing 40 is fitted to the outer surface of the slide rod sleeve 41 with a clearance; a plurality of axial slide rails 43-B are distributed on the outer surface of the slide sleeve 43, and the outer surface of the slide sleeve 43 and the plurality of slide rails 43-B are fitted to the light guiding tube frame with a clearance; the inner surface of the slide sleeve 43 is fitted to the outer surface of the first bearing 40 with an interference; the slide sleeve 43 has a protruding end fixedly connected to the moving rod of the linear motor 30; the slide rod 44 has one end fixedly connected to the bearing retaining ring 42 or the slide rod sleeve 41, and another end hinged to the first reflector mount 48 via the linkage mechanism 45.

Optionally, the light guiding tube 37 is positioned on the light guiding tube frame 1-1 connected to the base 1 via a second bearing 38. Specifically, the inner surface of the second bearing 38 is fitted to the outer wall of the light guiding tube 37 with an interference; the outer surface of the second bearings 38 is fitted to the light guiding tube frame 1-1 with an interference.

The 4DOF laser processing device above includes a linear motor 30 and a second transmission mechanism positioned on a base 1, and accordingly, in comparison with a 3DOF processing device, has a first reflector mount 48 that is rotatable around an axis of the hinge, which enables the working plane of the laser cutter to rotate around the axis of the hinge, thereby increasing the degrees of freedom of the laser cutter, expanding the application of the laser processing device and satisfying the need for a versatile processing device.

It should be noted that, in this example, a second linear motor directly drives the sliding of the slide sleeve; however, based on the example, those skilled in the art can easily implement the same function by replacing the linear motor with such equivalents as rotating motor + cylindrical cam, or rotating motor + slider-crank mechanism.

Embodiment 1 of the present invention

This embodiment provides a 5DOF laser processing device. The device can be basically the same as the 4DOF laser processing device described in Example 1, except that: the present embodiment additionally includes: a linear motor 30 and a second transmission mechanism positioned on the base 1. Specifically, the first reflector mount 48 is hinged to the end of the light guiding tube 37; the first reflector mount 48 is drivable by the linear motor 30 via the second transmission mechanism to rotate around an axis of the hinge under control.

In this embodiment, the rotating motor 8, the first transmission mechanism, the linear motor 30, the second transmission mechanism and structures that cooperate with these structures are collectively referred to as a 2DOF laser adjustment unit. That is, the 2DOF laser adjustment unit includes: a rotating motor 8 and related components, a second linear motor 30 and related components, and a first reflector mount 48 and related components. The rotating motor 8 realizes a 360-degree roll of the first reflector by a transmission mechanism (e.g., gear transmission, belt transmission, or steel wire transmission); the second linear motor realizes a certain degree (e.g., 0 to 110 degrees) of pitch of the first reflector by a four-bar linkage mechanism, thereby realizing a wide-range large-degree 2DOF position adjustment of the laser cutter.

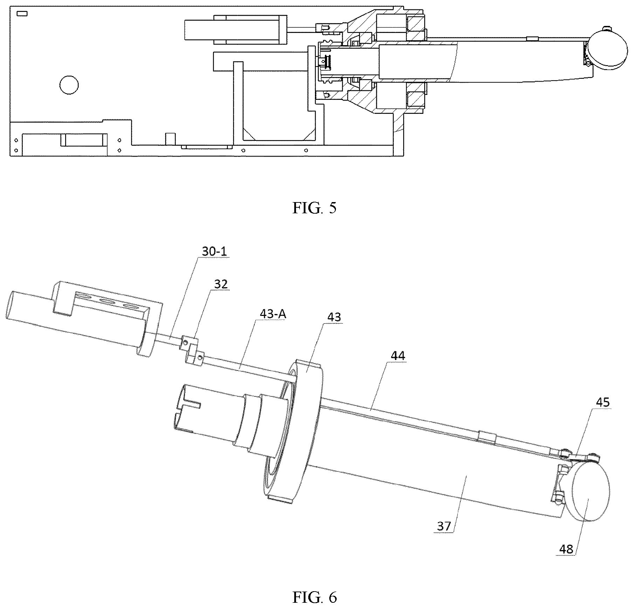

FIG. 4 is an overall structural view of a 2DOF laser adjustment unit according to an embodiment of the present invention. FIG. 5 is a cross-section view of a part of the 2DOF laser adjustment unit. The second linear motor 30 is fixed to a second linear motor mount 6. The second linear motor mount is connected to the base by a screw 6-1. The moving rod 30-1 of the second linear motor is connected to the bearing slide sleeve 43 by a rod connector 32, so that the second linear motor can drive the sliding of the bearing slide sleeve. The rotating motor 8 is fixed on a rotating motor mount 7, and the rotating motor mount is connected to the base by a screw 7-1. A driving wheel 33 is fixed to the output shaft of the rotating motor, and drives a driven wheel 34 to rotate via a transmission component (e.g., gear transmission, belt transmission, or steel wire transmission).

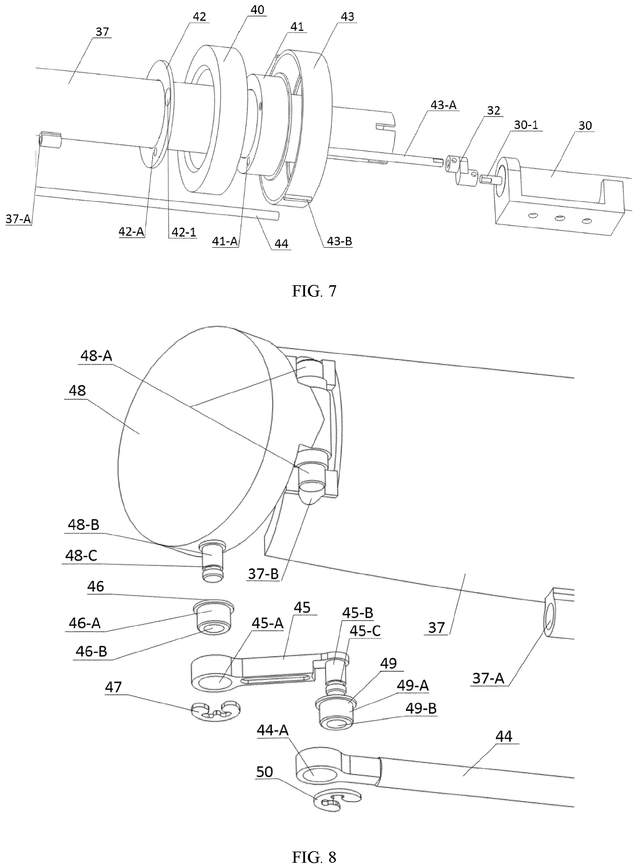

As shown in FIG. 6 and FIG. 7, pitch adjustment of the laser beam is realized by the rotation of the first reflector mount 48 around the axis 48-A, and the rotation around the axis 48-A is realized with a slider-crank mechanism. The slide sleeve 43 is engaged with a slide groove on the base by 43-B, so that the slide sleeve and the base can slide relative to each other. The protruding end 43-A of the slide sleeve passes through the base, and is fixedly connected to the moving rod of the second linear motor by a rod connector, so that the second linear motor drives the slide sleeve to slide relative to the base. The outer surface of the first bearing 40 is fitted to the slide sleeve, and the inner surface of the first bearing is fitted to the slide rod sleeve 41, so that the first bearing realizes the relative rotation between the slide sleeve and the slide rod sleeve. The slide rod 44 passes through the hole 37-A of the light guiding tube 37 and the hole 42-A of the first bearing retaining ring 42, and enters the hole 41-A of the slide sleeve. The slide rod is fixedly connected to the slide rod sleeve. The first bearing retaining ring is connected to the slide rod sleeve by a screw 42-1, to restrain the axial position of the first bearing.

As shown in FIG. 8, the slide rod hole 44-A is fitted to a surface 49-A of a first bearing shell 49; a first bearing shell hole 49-B is fitted to a surface 45-B of a connecting rod 45; a first retaining ring 50 is inserted in a connecting rod groove 45-C, thereby realizing the rotation of the slide rod relative to the connecting rod and restraining the axial position of the slide rod. A slide rod hole 45-A is fitted to a surface 46-A of a second bearing shell 46; a second bearing hole 46-B is fitted to a surface 48-B of the first reflector mount 48; a second retaining ring 47 is inserted in a first reflector mount groove 48-C, thereby realizing the rotation of the connecting rod relative to the first reflector mount. A first reflector mount shaft 48-A is fitted to a hole 37-B of the light guiding tube 37, thereby realizing the relative rotation between the first reflector mount and the light guiding tube.

As shown in FIG. 8, the relative rotation between the slide rod and the connecting rod is realized by a bearing shell; however, the structure shown in FIG. 8 is not the only implementation for the above relative rotation. For example, a rotary bearing (e.g., deep groove ball bearing) can realize the same function. In the embodiment according to FIG. 8, the pitch of the first reflector mount is realized by a slider-crank mechanism; in other embodiments, the same effect can be achieved using a rotating motor + wire transmission method.

Pitch adjustment of the laser cutter is realized by the following process. The moving rod of the second linear motor drives the slide sleeve to slide in the base groove 1-C, forming a sliding pair and driving the first bearing to slide. Because the first bearing is fixed to the slide rod sleeve by the first bearing retaining ring, the slide rod sleeve is also driven to slide. The slide rod sleeve (equivalently a slider), the connecting rod, the first reflector mount (equivalently a crank) and the light guiding tube form a slider-crank four-bar mechanism. Therefore, when the slide rod sleeve is driven to slide by the second linear motor, it drives the first reflector mount to rotate relative to the light guiding tube, thereby changing the pitch angle of the laser cutter.

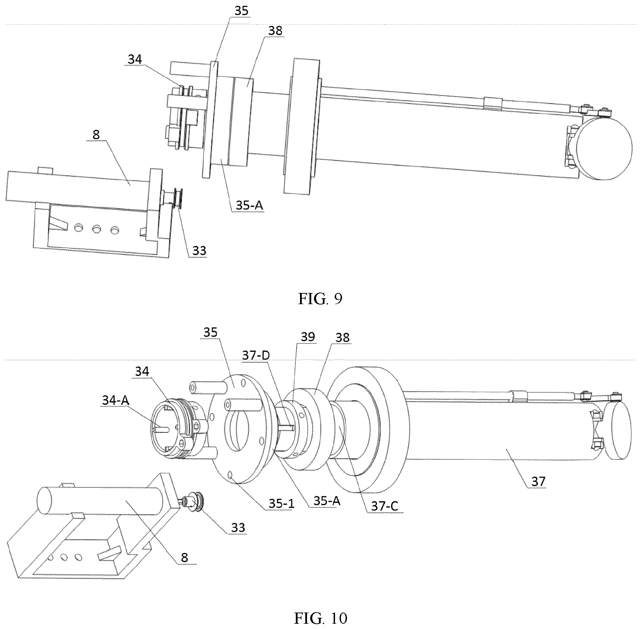

As shown in FIG. 9 and FIG. 10, an output shaft of the rotating motor 8 is connected to the driving wheel 33 by threads. The driving wheel drives the driven wheel 34 to rotate by a transmission mechanism (e.g., belt transmission, gear transmission, chain transmission, steel wire transmission). The driven wheel 34-A is fitted to a light guiding tube groove 37-D, and is fixed in the axial position by threads. The inner surface of the second bearing 38 is fitted to a light guiding tube surface 37-C, and the outer surface of the second bearing is fitted to a base 1-B. The second shaft retaining ring 39 bears against the inner surface of the second bearing and is fixed to the light guiding tube by threads. An end cap 35 is connected to the base by a screw 35-1, and has a protruding end 35-A pressed against the outer surface of the second bearing. Based on the design above, the light guiding tube is installed in the base via the second bearing and can rotate relative to the base; the second bearing retaining ring and the end cap limit the axial position of the second bearing, and limit the axial position of the light guiding tube. When the driven wheel is driven by the driving wheel to rotate, it drives the light guiding tube to rotate relative to the base through the cooperation between 34-A and 37-D, thereby causing the first reflector mount to roll and adjusting the rolling angle of the laser cutter.

In the 5DOF laser processing device above, the second linear motor 30 is fixed on the second linear motor mount 6; the second linear motor mount is connected to the base by a screw 6-1; and the moving rod 30-1 of the second linear motor is connected to the bearing slide sleeve 43 by the rod connector 32, so that the second linear motor can drive the bearing slide sleeve to slide. The rotating motor 8 is fixed on the rotating motor mount 7; the rotating motor mount is connected to the base by a screw 7-1; and the driving wheel 33 is fixed to the output shaft of the rotating motor, and drives the driven wheel 34 to rotate via a transmission mechanism (gear transmission, belt transmission, wire transmission, chain transmission, etc.). In this embodiment, the two degrees of freedom of the laser cutter can be adjusted independently without interfering with each other. When the light guiding tube rotates relative to the base, it drives the first reflector mount, the connecting rod, the slide rod, the slide rod sleeve and the inner surface of the first bearing to rotate together, and the second linear motor drives the slide sleeve, the second bearing, the slide rod sleeve and the slide rod on base grooves (i.e., grooves that match the slide rails 43-B), thereby realizing the rotation and rolling of the first reflector mount.

Embodiment 2, according to the present invention

This embodiment provides a Multi-DOF laser processing device. The device can be basically the same as in embodiment 1, except that: the present embodiment includes, on top of the 4DOF laser processing device or the 5DOF laser processing device, a sixth degree of freedom, by positioning the base 1 of the Multi-DOF laser processing device on a working platform that can be controlled to move in the axial direction of the light guiding tube.

Extension or contraction of the light guiding tube 37 is difficult, because the structure is complicated (decoupling requirement from the two degrees of freedom pitch and roll) and because it changes the position of the focal plane of the laser (Retaining the focal plane will need the lens to move). Instead, in this embodiment, the base 1 is positioned on a working platform that can be controlled to move in the axial direction of the light guiding tube 37, thereby realizing a sixth degree of freedom of laser cutter adjustment. A main object of this is to provide adjustment function when lens adjustment is not enough. With this embodiment, the position of the laser cutter can be made deeper.

Embodiment 3, according to the present invention

This embodiment provides a Multi-DOF laser processing device. The device can be basically the same as any one of Embodiments 1 to 2, except that: the present embodiment additionally includes: a laser processing monitoring unit; and the laser processing monitoring unit includes: a CCD camera frame (25, 26) positioned on the base, a CCD camera mounted on the CCD camera frame, a reflector and a beam combiner. Specifically, the beam combiner is positioned at a laser entering end of the light guiding tube so that the surface of the beam combiner is at a certain angle to the axial direction of the light guiding tube; the reflector is positioned on the base and cooperates with the beam combiner so that an auxiliary light irradiated on the processing surface can enter the CCD camera for imaging after reflections by the beam combiner and the reflector.

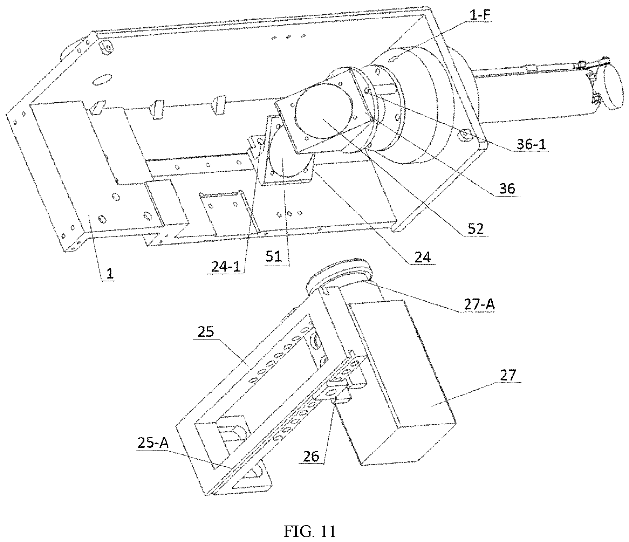

FIG. 11 is a structural view of a laser processing monitoring unit. A beam combiner 52 is installed on a beam combiner mount 36, and the beam combiner mount is connected to an end cap of the base by a screw 36-1. A second reflector 51 is installed on a second reflector mount 24, and the second reflector mount is connected to the base by a screw 24-1. A CCD camera connecting frame 26 is slidable in a groove 25-A of a CCD camera support frame 25, so that the position of the CCD camera 27 can be adjusted, thereby obtaining a clearer monitoring video. During laser processing, an auxiliary light (e.g., infrared, visible light) is irradiated on the processing surface. The auxiliary light is reflected by the first reflector (coated with a double-layer film that can reflect both the processing laser beam and the monitoring light), and passes through the light guiding tube. Then the auxiliary light is reflected by the beam combiner 52 (films different on either side, where the laser-facing side is coated with a film anti-reflective to laser while the auxiliary light facing side is coated with a film highly-reflective to the auxiliary light), and reflected by the second reflector 51, then enters an industrial lens 27 -A and finally hits the CCD camera 27. Through data transmission, the signal is transmitted to a receiving platform, where a monitoring software (window) displays real-time situation of the laser processing. Therefore, in the event of an emergency, an operator can cut the power and laser off to protect processing material and equipment.

Embodiment 4, according to the present invention

This embodiment provides a Multi-DOF laser processing device. The device can be basically the same as any one of Embodiments 1 to 3, except that: the present embodiment additionally includes: an electromechanical control system; and the electromechanical control system is electrically connected to a 3DOF processing unit, a rotating motor, a linear motor, a CCD camera and a working platform, to realize the control or monitoring function.

The effects of the degrees of freedom laser cutter adjustments according to the embodiments above will be described below with reference to the accompanying drawings.

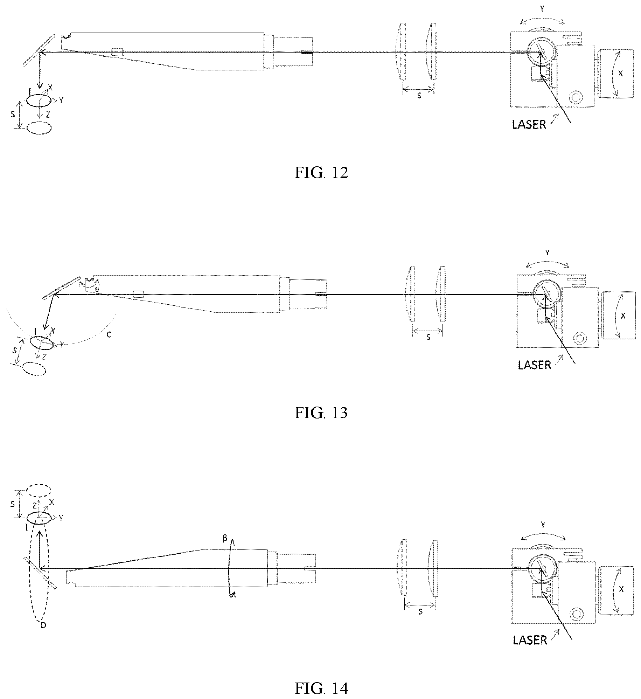

FIG. 12 is a schematic diagram illustrating 3DOF laser processing. The XY mirror galvanometers control the X-Y two-dimensional plane scanning; lens movement controls Z direction stepping. The laser beam enters through a light entering port of the base, and gets reflected by the XY mirror galvanometers, focused by the lens, then reflected by the first reflector to a surface I of the object to be processed. According to a laser processing path designed by a CAD/CAM system, the XY mirror galvanometers and lens are precisely controlled to shape the object into a predefined shape.

FIG. 13 is a schematic diagram illustrating pitch adjustment of the 2DOF laser processing. According to the relative position of the object to be processed to the device, the pitch angle of the first reflector mount is adjusted so that the laser beam can precisely reach the target position of the object to be processed. FIG. 13 shows one of the cases where the laser beam can reach any location on the curved surface C as the pitch angle changes, thereby meeting different needs.

FIG. 14 is a schematic diagram illustrating roll adjustment of the 2DOF laser processing. According to the relative position of the object to be processed to the device, the roll angle of the first reflector mount is adjusted so that the laser beam can precisely reach the target position of the object to be processed. FIG. 14 shows one of the cases where the laser beam can reach any location on the curved surface D as the pitch angle changes, thereby meeting different needs.

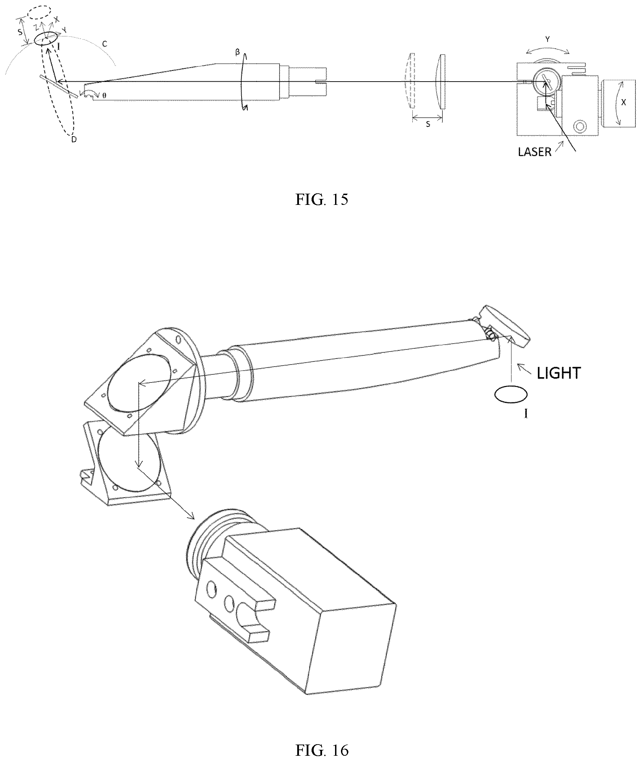

FIG. 15 is a schematic diagram illustrating pitch-plus-roll adjustment of the 2DOF laser processing. According to the relative position of the object to be processed to the device, the pitch angel and roll angle of the first reflector mount are adjusted so that the laser beam can precisely reach the target position of the object to be processed.

FIG. 16 is a schematic diagram illustrating an optical path for laser processing monitoring. The auxiliary light is reflected from the surface of the object to be processed, and enters the CCD camera through the first reflector, the beam combiner and the second reflector. The position of the CCD camera can be adjusted according to the position of the exit auxiliary light, so that CCD imaging is clear and complete for real-time monitoring of the laser processing process, and when an emergency occurs, the operator can cut the power and laser off.

In summary, the Multi-DOF laser processing device according to the embodiment of the present invention can achieve high-stability non-vibration high-precision cutting of diseased tissues of any shape, and surface modification and precise preparation of materials. The Multi-DOF laser processing device is equipped with a laser, and realizes three-dimensional spot processing by mirror galvanometer + lens. The Multi-DOF laser processing device uses a two-dimensional laser adjustment unit to realize a 360° rotation and a 0-110° pitch of the laser cutter, and uses an additional 1DOF moving platform on the base to realize wide-range adjustment of the position of the laser cutter. Each degree of freedom is provided with a position sensor for closed-loop feedback, in order to achieve high-speed high-precision high-efficiency processing and cutting.

Each of the following advantageous effects can be realized by at least one of the embodiments of the present invention.
1. Wide application range: The device is applicable for surgical operations (especially small and deep cavity) in various fields such as oral cavity, throat, ophthalmology, orthopedics and surgery, as well as industrial fields such as surface modification, treatment and precision preparation.
2. Highly dexterous: the device is small in size, but integrated with an optical system including mirror galvanometers, lens and reflectors and an electromechanical system including a plurality of motors that control the optical system as well as a real-time monitoring apparatus. The device can control 6DOF movement of the laser, thereby realizing cutting in any direction at any diseased tissue.
3. Precise and safe: The device can precisely control 6DOF movement of the laser and 6DOF laser cutting, thereby realizing high-precision cutting of diseased tissues. The device is also provided with a monitoring device and a high-precision position sensor for safety control.

Preferred embodiments of the present invention are described above for illustrative purposes only and shall not be construed as limiting. Those skilled in the art would understand that various modifications and changes such as changing the pitch angle to 0-120° degrees shall fall within the scope of the present invention as defined in the appended claims.

## Claims

1. A multiple degrees of freedom (Multi-DOF) laser processing device, comprising a base (1), and a three degrees of freedom (3DOF) laser processing unit (10, 11, 14) and a light guiding tube (37) positioned on the base, the light guiding tube having an end provided with a first reflector mount (48), the 3DOF laser processing unit being configured to adjust a focal length of a laser to adjust a focal plane of a laser cutter and control movement of the laser cutter on the focal plane, **characterized in that**, the Multi-DOF laser processing device further comprises: a rotating motor (8) and a first transmission mechanism positioned on the base (1), wherein the light guiding tube (37) is rotatably positioned on a light guiding tube frame (1-1) that is connected to the base; the light guiding tube (37) is drivable by the rotating motor (8) via the first transmission mechanism, to rotate on the light guiding tube frame (1-1) under control; wherein the Multi-DOF laser processing device further comprises: a linear motor (30) Z and a second transmission mechanism positioned on the base (1), and the first reflector mount (48) is hinged to the end of the light guiding tube (37); the first reflector mount (48) is drivable by the linear motor (30) via the second transmission mechanism to rotate around an axis of the hinge under control.

2. The Multi-DOF laser processing device according to claim 1, wherein the first transmission mechanism comprises: a driving wheel (33) positioned on an output shaft of the rotating motor, a driven wheel (34) positioned on the light guiding tube (37), and a transmission component for transmitting the rotation of the driving wheel to the driven wheel.

3. The Multi-DOF laser processing device according to claim 2, wherein the driven wheel (34) has a diameter greater than that of the driving wheel (33).

4. The Multi-DOF laser processing device according to claim 1, wherein the second transmission mechanism comprises: a slide sleeve (43), a first bearing (40), a slide rod sleeve (41), a bearing retaining ring (42), a slide rod (44) and a linkage mechanism (45), and the slide rod sleeve (41) is fixed to the bearing retaining ring (42), an inner surface of the slide rod sleeve (41) and an inner surface of the bearing retaining ring (42) are both fitted to the light guiding tube (37) with a clearance; an inner surface of the first bearing (40) is fitted to an outer surface of the slide rod sleeve (41) with a clearance; a plurality of axial slide rails (43-B) are distributed on an outer surface of the slide sleeve (43), and the outer surface of the slide sleeve (43) and the plurality of slide rails (43-B) are fitted to the light guiding tube frame (1-1) with a clearance; an inner surface of the slide sleeve (43) is fitted to an outer surface of the first bearing (40) with an interference; the slide sleeve (43) has a protruding end (43-A) fixedly connected to a moving rod (30-A) of the linear motor; the slide rod (44) has one end fixedly connected to the bearing retaining ring (42) or the slide rod sleeve (41), and another end hinged to the first reflector mount (48) via the linkage mechanism.

5. The Multi-DOF laser processing device according to claim 1, wherein the light guiding tube (37) is positioned on the light guiding tube frame (1-1) connected to the base via a second bearing (38), and an inner surface of the second bearing (38) is fitted to an outer wall of the light guiding tube (37) an interference; an outer surface of the second bearings (38) is fitted to the light guiding tube frame (1-1) with an interference.

6. The Multi-DOF laser processing device according to claim 1, wherein the base (1) is positioned on a working platform that is controllable to be moved in an axial direction of the light guiding tube (37).

7. The Multi-DOF laser processing device according to any one of claims 1 to 6, wherein the Multi-DOF laser processing device further comprises: a laser processing monitoring unit, and the laser processing monitoring unit comprises: a CCD camera frame (25) positioned on the base, a CCD camera (27) mounted on the CCD camera frame, a reflector (51) and a beam combiner (52), and the beam combiner (52) is positioned at a laser entering end of the light guiding tube (37) so that a surface of the beam combiner is at a certain angle to an axial direction of the light guiding tube; the reflector (51) is positioned on the base and cooperates with the beam combiner so that an auxiliary light irradiated on the processing surface can enter the CCD camera (27) for imaging after reflections by the beam combiner (52) and the reflector (51).

8. The Multi-DOF laser processing device according to claim 7, wherein the beam combiner (52) has a laser-facing side coated with a film anti-reflective to laser and an auxiliary light-facing side coated with a film highly-reflective to the auxiliary light.

9. The Multi-DOF laser processing device according to any one of claims 1 to 8, wherein the Multi-DOF laser processing device further comprises an electromechanical control system, the electromechanical control system being electrically connected to the 3DOF processing unit, the rotating motor (8), the linear motor (30), the CCD camera (27) and the working platform, to realize control or monitoring function.

## Patentansprüche

1. Laserbearbeitungsvorrichtung mit mehreren Freiheitsgraden (Multi-DOF), umfassend eine Basis (1) und eine Laserbearbeitungseinheit (10, 11, 14) mit drei Freiheitsgraden (3DOF) und einen Lichtleitstab (37), der auf der Basis positioniert ist, wobei der Lichtleitstab ein Ende aufweist, das mit einem ersten Reflektorhalter (48) versehen ist, wobei die 3DOF-Laserbearbeitungseinheit dazu konfiguriert ist, eine Brennweite eines Lasers einzustellen, um eine Brennebene eines Laserschneiders einzustellen und die Bewegung des Laserschneiders auf der Brennebene zu steuern, **dadurch gekennzeichnet, dass** die Multi-DOF-Laserbearbeitungsvorrichtung ferner Folgendes umfasst: einen Drehmotor (8) und einen ersten Übertragungsmechanismus, der auf der Basis (1) positioniert ist, wobei der Lichtleitstab (37) rotierbar auf einem Lichtleitstabrahmen (1-1) positioniert ist, der mit der Basis verbunden ist;
wobei der Lichtleitstab (37) durch den Drehmotor (8) über den ersten Übertragungsmechanismus antreibbar ist, um kontrolliert auf dem Lichtleitstabrahmen (1-1) zu rotieren;
wobei die Multi-DOF-Laserbearbeitungsvorrichtung ferner Folgendes umfasst: einen Linearmotor (30) und einen zweiten Übertragungsmechanismus, der auf der Basis (1) positioniert ist, und wobei der erste Reflektorhalter (48) gelenkig mit dem Ende des Lichtleitstabs (37) verbunden ist; wobei der erste Reflektorhalter (48) von dem Linearmotor (30) über den zweiten Übertragungsmechanismus antreibbar ist, um kontrolliert um eine Achse des Gelenks zu rotieren.

2. Multi-DOF-Laserbearbeitungsvorrichtung nach Anspruch 1, wobei der erste Übertragungsmechanismus Folgendes umfasst: ein antreibendes Rad (33), das auf einer Ausgangswelle des Drehmotors positioniert ist, ein angetriebenes Rad (34), das auf dem Lichtleitstab (37) positioniert ist, und eine Übertragungskomponente zum Übertragen der Rotation des antreibenden Rads auf das angetriebene Rad.

3. Multi-DOF-Laserbearbeitungsvorrichtung nach Anspruch 2, wobei das angetriebene Rad (34) einen Durchmesser aufweist, der größer als der des antreibenden Rads (33) ist.

4. Multi-DOF-Laserbearbeitungsvorrichtung nach Anspruch 1, wobei der zweite Übertragungsmechanismus Folgendes umfasst: eine Gleithülse (43), ein erstes Lager (40), eine Gleitstangenhülse (41), einen Lagerhaltering (42), eine Gleitstange (44) und einen Verbindungsmechanismus (45), und wobei die Gleitstangenhülse (41) an dem Lagerhaltering (42) befestigt ist, wobei eine Innenfläche der Gleitstangenhülse (41) und eine Innenfläche des Lagerhalterings (42) beide mit einem Abstand an dem Lichtleitstab (37) angebracht sind;
wobei eine Innenfläche des ersten Lagers (40) mit einem Abstand an einer Außenfläche der Gleitstangenhülse (41) angebracht ist;
wobei eine Vielzahl von axialen Gleitschienen (43-B) auf einer Außenfläche der Gleithülse (43) verteilt ist und die Außenfläche der Gleithülse (43) und die Vielzahl von Gleitschienen (43-B) mit einem Abstand an dem Lichtleitstabrahmen (1-1) angebracht sind;
wobei eine Innenfläche der Gleithülse (43) mit einer Interferenz an einer Außenfläche des ersten Lagers (40) angebracht ist;
wobei die Gleithülse (43) ein vorstehendes Ende (43-A) aufweist, das fest mit einer sich bewegenden Stange (30-A) des Linearmotors verbunden ist;
wobei die Gleitstange (44) ein Ende, das fest mit dem Lagerhaltering (42) oder der Gleitstangenhülse (41) verbunden ist, und ein anderes Ende, das über den Verbindungsmechanismus gelenkig mit dem ersten Reflektorhalter (48) verbunden ist, aufweist.

5. Multi-DOF-Laserbearbeitungsvorrichtung nach Anspruch 1, wobei der Lichtleitstab (37) auf dem Lichtleitstabrahmen (1-1) positioniert ist, der über ein zweites Lager (38) mit der Basis verbunden ist, und eine Innenfläche des zweiten Lagers (38) mit einer Interferenz an einer Außenwand des Lichtleitstabs (37) angebracht ist;
wobei eine Außenfläche des zweiten Lagers (38) mit einer Interferenz an dem Lichtleitstabrahmen (1-1) angebracht ist.

6. Multi-DOF-Laserbearbeitungsvorrichtung nach Anspruch 1, wobei die Basis (1) auf einer Arbeitsplattform positioniert ist, die steuerbar ist, um in einer axialen Richtung des Lichtleitstabs (37) bewegt zu werden.

7. Multi-DOF-Laserbearbeitungsvorrichtung nach einem der Ansprüche 1 bis 6, wobei die Multi-DOF-Laserbearbeitungsvorrichtung ferner Folgendes umfasst: eine Laserbearbeitungsüberwachungseinheit, und wobei die Laserbearbeitungsüberwachungseinheit Folgendes umfasst: einen CCD-Kamerarahmen (25), der auf der Basis positioniert ist, eine CCD-Kamera (27), die auf dem CCD-Kamerarahmen montiert ist, einen Reflektor (51) und einen Strahlkombinierer (52), und wobei der Strahlkombinierer (52) an einem Lasereintrittsende des Lichtleitstabs (37) positioniert ist, sodass eine Oberfläche des Strahlkombinierers in einem bestimmten Winkel zu einer axialen Richtung des Lichtleitstabs ist;
wobei der Reflektor (51) auf der Basis positioniert ist und mit dem Strahlkombinierer zusammenwirkt, sodass ein Zusatzlicht, das auf die Verarbeitungsoberfläche gestrahlt wird, in die CCD-Kamera (27) zum Abbilden nach Reflexionen durch den Strahlkombinierer (52) und den Reflektor (51) eintreten kann.

8. Multi-DOF-Laserbearbeitungsvorrichtung nach Anspruch 7, wobei der Strahlkombinierer (52) eine dem Laser zugewandte Seite, die mit einem Film beschichtet ist, der für Laser antireflektierend ist, und eine dem Zusatzlicht zugewandte Seite, die mit einem Film beschichtet ist, der für das Zusatzlicht hochreflektierend ist, aufweist.

9. Multi-DOF-Laserbearbeitungsvorrichtung nach einem der Ansprüche 1 bis 8, wobei die Multi-DOF-Laserbearbeitungsvorrichtung ferner ein elektromechanisches Steuersystem umfasst, wobei das elektromechanische Steuersystem elektrisch mit der 3DOF-Bearbeitungseinheit, dem Drehmotor (8), dem Linearmotor (30), der CCD-Kamera (27) und der Arbeitsplattform verbunden ist, um eine Steuer- oder Überwachungsfunktion zu realisieren.

## Revendications

1. Dispositif de traitement au laser à multiples degrés de liberté (Multi-DOF), comprenant une base (1), et une unité de traitement au laser à trois degrés de liberté (3DOF) (10, 11, 14), et un tube de guidage de lumière (37) positionné sur la base, le tube de guidage de lumière présentant une extrémité dotée d'un premier support de réflecteur (48), l'unité de traitement au laser 3DOF étant configurée de manière à ajuster une longueur focale d'un laser pour ajuster un plan focal d'un découpeur au laser et commander le mouvement du découpeur au laser sur le plan focal, **caractérisé en ce que** le dispositif de traitement au laser Multi-DOF comprend en outre :
un moteur rotatif (8) et un premier mécanisme de transmission positionné sur la base (1), dans lequel le tube de guidage de lumière (37) est positionné de manière rotative sur un cadre de tube de guidage de lumière (1-1) qui est connecté à la base ;
le tube de guidage de lumière (37) peut être entraîné par le moteur rotatif (8) par l'intermédiaire du premier mécanisme de transmission, afin de tourner sur le cadre de tube de guidage de lumière (1-1) sous contrôle ;
dans lequel le dispositif de traitement au laser Multi-DOF comprend en outre : un moteur linéaire (30) et un second mécanisme de transmission positionné sur la base (1), et le premier support de réflecteur (48) est articulé à l'extrémité du tube de guidage de lumière (37) ;
le premier support de réflecteur (48) peut être entraîné par le moteur linéaire (30) par l'intermédiaire du second mécanisme de transmission pour tourner autour d'un axe de la charnière sous contrôle.

2. Dispositif de traitement au laser Multi-DOF selon la revendication 1, dans lequel le premier mécanisme de transmission comprend : une roue d'entraînement (33) positionnée sur un arbre de sortie du moteur rotatif, une roue entraînée (34) positionnée sur le tube de guidage de lumière (37), et un composant de transmission pour transmettre la rotation de la roue d'entraînement à la roue entraînée.

3. Dispositif de traitement au laser Multi-DOF selon la revendication 2, dans lequel la roue entraînée (34) présente un diamètre supérieur à celui de la roue d'entraînement (33).

4. Dispositif de traitement au laser Multi-DOF selon la revendication 1, dans lequel le second mécanisme de transmission comprend : un manchon coulissant (43), un premier palier (40), un manchon de tige coulissante (41), une bague de retenue de palier (42), une tige coulissante (44) et un mécanisme de liaison (45) ; et
le manchon de tige coulissante (41) est fixé à la bague de retenue de palier (42), une surface intérieure du manchon de tige coulissante (41) et une surface intérieure de la bague de retenue de palier (42) sont toutes deux ajustées au tube de guidage de lumière (37) avec un jeu ;
une surface intérieure du premier palier (40) est ajustée sur une surface extérieure du manchon de tige coulissante (41) avec un jeu ;
une pluralité de glissières axiales (43-B) est répartie sur une surface extérieure du manchon coulissant (43), et la surface extérieure du manchon coulissant (43) et la pluralité de glissières (43-B) est ajustée au cadre de tube de guidage de lumière (1-1) avec un jeu ;
une surface intérieure du manchon coulissant (43) est ajustée à une surface extérieure du premier palier (40) avec un serrage ;
le manchon coulissant (43) présente une extrémité en saillie (43-A) reliée de manière fixe à une tige mobile (30-A) du moteur linéaire ;
la tige coulissante (44) présente une extrémité reliée de manière fixe à la bague de retenue de palier (42) ou au manchon de tige coulissante (41), et une autre extrémité articulée au premier support de réflecteur (48) par l'intermédiaire du mécanisme de liaison.

5. Dispositif de traitement au laser Multi-DOF selon la revendication 1, dans lequel le tube de guidage de lumière (37) est positionné sur le cadre de tube de guidage de lumière (1-1) relié à la base par l'intermédiaire d'un second palier (38) ; et
une surface intérieure du second palier (38) est ajustée à une paroi extérieure du tube de guidage de lumière (37) avec un serrage ;
une surface extérieure du second palier (38) est ajustée au cadre de tube de guidage de lumière (1-1) avec un serrage.

6. Dispositif de traitement au laser Multi-DOF selon la revendication 1, dans lequel la base (1) est positionnée sur une plateforme de travail qui peut être commandée en vue d'être déplacée dans une direction axiale du tube de guidage de lumière (37).

7. Dispositif de traitement au laser Multi-DOF selon l'une quelconque des revendications 1 à 6, dans lequel le dispositif de traitement au laser Multi-DOF comprend en outre : une unité de surveillance de traitement au laser, et l'unité de surveillance de traitement au laser comprend : un cadre de caméra CCD (25) positionné sur la base, une caméra CCD (27) montée sur le cadre de caméra CCD, un réflecteur (51) et un combineur de faisceau (52), et dans lequel le combineur de faisceau (52) est positionné à une extrémité d'entrée de laser du tube de guidage de lumière (37) de sorte qu'une surface du combineur de faisceau est à un angle donné par rapport à une direction axiale du tube de guidage de lumière ; et
le réflecteur (51) est positionné sur la base et coopère avec le combineur de faisceau de sorte qu'une lumière auxiliaire irradiée sur la surface de traitement peut entrer dans la caméra CCD (27) pour une imagerie après des réflexions par le combineur de faisceau (52) et le réflecteur (51).

8. Dispositif de traitement au laser Multi-DOF selon la revendication 7, dans lequel le combineur de faisceau (52) présente un côté faisant face au laser enduit d'un film anti-réfléchissant au laser et un côté faisant face à la lumière auxiliaire enduit d'un film hautement réfléchissant à la lumière auxiliaire.

9. Dispositif de traitement au laser Multi-DOF selon l'une quelconque des revendications 1 à 8, dans lequel le dispositif de traitement au laser Multi-DOF comprend en outre un système de commande électromécanique, le système de commande électromécanique étant électriquement connecté à l'unité de traitement 3DOF, au moteur rotatif (8), au moteur linéaire (30), à la caméra CCD (27) et à la plateforme de travail, pour réaliser une fonction de commande ou de surveillance.
